# NOUVEAU FASCICULE DE BREVET EUROPEEN

(11) **EP 0 634 162 B2**
(45) Date de publication et mention de la décision concernant l'opposition: **22.07.2009**
(45) Mention de la délivrance du brevet: 04.11.1998
(21) Numéro de dépôt: 94401610.4
(22) Date de dépôt: 12.07.1994
(51) Int. Cl.: A61K 8/41, A61Q 5/10

(54) **Composition de teinture d'oxydation des fibres kératiniques comprenant un para-aminophénol, un méta-aminophénol et une métaphénylènediamine**
Oxidationsfärbemittel für keratinische Fasern enthaltend ein para-Aminophenol, ein meta-Aminophenol und ein Metaphenylendiamin
Dyeing composition for keratinous fibers containing a para-aminophenol, a meta-aminophenol and a metaphenylendiamine

(30) Priorité: 13.07.1993 FR 9308614
(43) Date de publication de la demande: 18.01.1995
(73) Titulaire: L'ORÉAL, 75008 Paris (FR)
(72) Inventeur: Cotteret, Jean, F-78480 Verneuil-sur-Seine (FR); Audousset, Marie Pascale, F-92300 Levallois-Perret (FR); Lagrange, Alain, F-77700 Coupvray (FR); Vandenbosche, Jean-Jacques, F-93279 Sevran (FR)
(74) Mandataire: Bulle, Françoise

(56) Documents cités:
- EP-A- 0 252 351
- EP-A- 0 459 900
- DE-A- 2 737 138
- DE-A1- 3 125 705
- FR-A- 2 421 870
- CHEMICAL ABSTRACTS, Columbus, Ohio, US; EXTRAIT D'UNE BASE DE DONNÉES DE LA SOCIÉTE WELLA AG: 'concernant le produit "Haarpur"'
- CHEMICAL ABSTRACTS, Columbus, Ohio, US; EXTRAIT D 'UNE BASE DE DONNÉES DE LA SOCIÉTÉ WELLA CONCERNANT: 'le produit "Lehmannblau"'
- CHEMICAL ABSTRACTS, Columbus, Ohio, US; EXTRAIT D'UNE BASE DE DONNÉES DE LA SOCIÉTÉ WELLA AG: 'concernant le produit "HC Blue AC"'
- CHEMICAL ABSTRACTS, Columbus, Ohio, US; (D3A/1) ET (D3A/2) EXTRAITS D'UN REGISTRE DE PRODUCTION: 'du produit Koleston 2000 Nuance 6/45'
- CHEMICAL ABSTRACTS, Columbus, Ohio, US; (D4C) ET (D4D) COPIES DE FACTURES CONCERNANT, ENTRE AUTRES: 'des produits Koleston 2000'
- CHEMICAL ABSTRACTS, Columbus, Ohio, US; BON DE LIVRAISON CONCERNANT, ENTRE AUTRES DES PRODUITS: 'Koleston 2000'
- CHEMICAL ABSTRACTS, Columbus, Ohio, US; SICHERHEITDATENBLATT DE GRAFOX CHEMIE VERTRIEBS GMBH: 'concernant le produit "Haarpur"'
- CHEMICAL ABSTRACTS, Columbus, Ohio, US; (D8C) COPIE D 'UN EMBALLAGE ET COPIES D'UN TUBE DE PRODUIT: 'Koleston 2000 6/45 portant le numéro de charge 87001'
- CHEMICAL ABSTRACTS, Columbus, Ohio, US; COPIE D'UN EXTRAIT DE LA BASE DE DONNÉES STN CONCERNANT: 'le 2-méthyl-5-aminophénol'
- CHEMICAL ABSTRACTS, Columbus, Ohio, US; COPIE D'UN EXTRAIT DE LA BASE DE DONNÉES STN CONCERNANT: 'le 2-méthyl-4-aminophénol'

## Description

La présente invention est relative à une composition de teinture des fibres kératiniques, et en particulier des fibres kératiniques humaines, comprenant, en association, au moins un para-aminophémol, au moins un 5-aminophénol 2-substitué et au moins une métaphénylèmediamine, et au procédé de teinture utilisant une telle composition et mettant en oeuvre la révélation par un agent oxydant.

Il est connu de teindre les fibres kératiniques et en particulier les cheveux humains, avec des compositions tinctoriales contenant des précurseurs de colorants d'oxydation, en particulier des ortho- ou paraphénylènediamines, des ortho- ou para-aminophénols, généralement appelés "bases d'oxydation", et des coupleurs encore appelés modificateurs de coloration, plus particulièrement des méta-phénylènediamines aromatiques, des méta-aminophénols et des méta-diphénols, qui permettent de modifier et d'enrichir de reflets les colorations "de fond" obtenues par les produits de condensation des bases d'oxydation.

On recherche, dans le domaine de la teinture capillaire d'oxydation, des précurseurs de colorants d'oxydation ainsi que des coupleurs qui permettent de conférer aux cheveux une coloration ayant une résistance satisfaisante à la lumière, aux lavages, aux intempéries, à la transpiration et aux différents traitements que peuvent subir les cheveux, et d'obtenir un large éventail de nuances de coloration.

Le 3-méthyl para-aminophénol ainsi que son utilisation dans des compositions tinctoriales pour fibres kératiniques en association avec le 2-méthyl 5-aminophénol à titre de coupleur et la para-phénylènediamine ou le 2,5-diaminotoluène, sont connus et décrits dans le brevet US-4.883.656.

Le brevet FR-A-2 421 870 décrit des métaphénylènediamines à titre de coupleurs et leur utilisation dans des compositions tinctoriales pour fibres kératiniques en association avec des précurseurs de colorants d'oxydation classiques tels que les para-phénylènediamines et les para-aminophénols, notamment la paraphénylènediamine, la para-toluylènediamine et le para-aminophénol et d'autres coupleurs connus du type méta-aminophénol.

Cependant, de telles associations ne procurent pas, après application sur les fibres kératiniques, une coloration suffisamment résistante.

La demanderesse vient de découvrir, ce qui fait l'objet de l'invention, que l'utilisation du 3-méthyl para-aminophénol, du 2-méthyl para-aminophénol et/ou du 2-hydroxyméthyl para-aminophénol à titre de précurseurs de colorants d'oxydation, en association avec au moins un 5-aminophériol substitué en position 2 à titre de coupleur, choisi parmi les 2-méthyl 5-aminophénols de formule (I) définie ci-après, et au moins une métaphénylènediamine à titre de coupleur additionnel, permet d'obtenir, en présence d'un agent oxydant, en milieu acide ou alcalin, après application sur les fibres kératiniques et en particulier les cheveux humains, des colorations aux nuances chaudes et cuivrées et présentant une bonne résistance à la lumière, aux lavages, aux intempéries, à la transpiration et aux différents traitements que peuvent subir les cheveux. La résistance à la transpiration est particulièrement remarquable et supérieure à celle de l'état de la technique.

La présente invention a donc pour objet une composition de teinture des fibres kératiniques, en particulier des fibres kératiniques humaines telles que les cheveux, comprenant, dans un milieu approprié pour la teinture :
- au moins un précurseur de colorant d'oxydation choisi parmi le 3-méthyl para-aminophénol, le 2-méthyl para-aminophénol et le 2-hydroxyméthyl para-aminaphénol, et leurs sels d'addition avec un acide:
- au moins un coupleur choisi parmi les 2-méthyl 5-aminophénols de formule (I) ci-après : 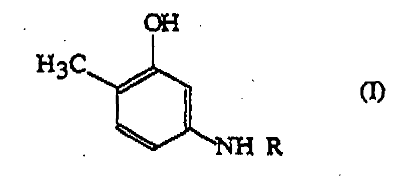 dans laquelle R désigne un radical méthyle ou éthyle, ou un groupement γ-hydroxypropyle, ainsi que leurs sels d'addition avec un acide: et
- au moins, à titre de coupleur additionnel, une métaphénylènediamine de formule (II) définie ci-après, ou l'un de ses sels d'addition avec un acide.

L'invention a également pour objet un agent de teinture à plusieurs composants, dont le premier composant contient les précurseurs de colorants d'oxydation et les coupleurs définis ci-dessus et le deuxième composant un agent oxydant.

Un autre objet de l'invention porte sur la composition prête à l'emploi, contenant les différents agents utilisés pour la teinture des fibres kératiniques définis ci-dessus et un agent oxydant, en milieu alcalin ou acide.

L'invention vise également un procédé de teinture des fibres kératiniques, et en particulier des fibres kératiniques humaines telles que les cheveux, consistant à appliquer sur ces fibres :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide:
- au moins un coupleur choisi parmi les 2-méthyl 5-aminophénols de formule (I) ci-dessus, ainsi que leurs sels d'addition avec un acide;
- au moins, à titre de coupleur additionnel, une métaphénylènediamine de formule (II) ou l'un de ses sels d'addition avec un acide:
la couleur étant révélée à pH acide ou alcalin, à l'aide d'un agent oxydant.

Selon l'invention et parmi les précurseurs de type para ci-dessus, le 3-méthyl p-aminophénol est préféré.

Les métaphénylènediamines qui sont utilisables selon l'invention, répondent à la formule (II) suivante : 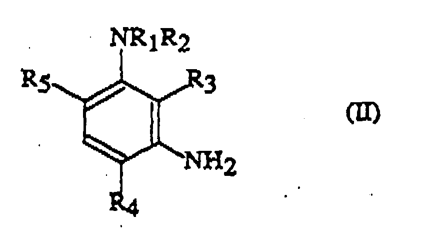 dans laquelle :
R₁ et R₂ désignent, indépendamment l'un de l'autre, un atome d'hydrogène ou un groupement alkyle en C₁-C₄ ou hydroxyalkyle en C₁-C₄;
R₃ désigne un atome d'hydrogène ou un groupement alkyle ou alcoxy en C₁-C₄:
R₄ désigne un atome d'hydrogène ou un groupement alkyle en C₁-C₄ ou hydroxyalcoxy en C₁-C₄, ou alcoxy en C₁-C₄:
R₅ désigne un atome d'hydrogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, un atome d'halogène, ou un groupement carboxyalcoxy en C₁-C₄, 2',4'-diaminophénoxyalcoxy en C₁-C₄, ou aminoalcoxy en C₁-C₄;
sous réserve que si R₅ désigne un groupement carboxyalcoxy ou 2',4-diaminophénoxyalcoxy, alors R₁, R₂, R₃ et R₄ désignent un atome d'hydrogène, si R₅ désigne un groupement alkyle en C₁-C₄ et R₁, R₂ et R₃ désignent un atome d'hydrogène, R₄ ne désigne pas un groupement alcoxy en C₁-C₄ ou hydroxyalcoxy en C₁-C₄, et si R₁, R₂ et R₄ désignent un atome d'hydrogène, R₃ et P₅ ne désignent pas simultanément un radical méthoxy.

Ces composés peuvent être utilisés sous forme libre ou salifiée.

Selon l'invention, on préfère, parmi les métaphénylènediamines de formule (II), celles pour lesquelles les groupements R₁ à R₄ désignent un atome d'hydrogène et R₅ désigne le groupement hydroxyalcoxy en C₁-C₄ ou polyhydroxyalcoxy en C₂-C₄,

Parmi les radicaux hydroxyalcoxy en C₁-C₄, on préfère plus particulièrement le radical β-hydroxyéthyloxy,

Parmi les radicaux polyhydroxyalcoxy en C₂-C₄, on préfère plus particulièrement le radical 1,2-dihydroxypropyloxy.

Parmi les composés de formule (II) préférés, on peut citer :
- le 1-β-hydroxyéthyloxy 2,4-diaminobenzène,
- le 1-(1,2-dihydroxypropyloxy)2,4-diaminobenzène,
- le 1-[2',4'-diaminophénoxypropyloxy]2.4-diaminobenzéne,
- le 1-méthoxy 2-amino 4-β-hydroxyéthylaminobenzène,
- l'acide 2,4-diaminaphénoxyacétique.
- le 4,6-bis-(2-hydroxyéthyloxy)1,3-diaminobenzène,
ainsi que leurs sels d'addition avec un acide.

Selon le procédé conforme à l'invention, on applique sur les fibres kératiniques humaines, au moins une composition (A) contenant dans un milieu approprié pour la teinture :
- au moins un précurseur de colorant d'oxydation choisi parmi le 3-méthyl para-aminophénol, le 2-méthyl para-aminophénol, le 2-hydroxyméthyl para-aminophénol, et leurs sels;
- au moins un coupleur choisi parmi les 2-méthyl 5-aminophénols de formule (I) ci-dessus, ainsi que leurs sels:
- au moins une métaphénylèmediamine de formule (II) ci-dessus, ou l'un de ses sels,
la couleur étant révélée en milieu acide ou alcalin. à l'aide d'un agent oxydant ajouté juste au moment de l'emploi à la composition (A) ou présent dans une composition (B) appliquée simultanément ou séquentiellement de façon séparée,

L'invention a également pour objet des dispositifs de teinture ou "kits", à plusieurs compartiments, permettant de mettre en oeuvre le procédé indiqué ci-dessus.

Un tel kit de teinture comporte au moins deux compartiments, dont le premier renferme la composition (A) telle que définie ci-dessus et le second renferme la composition (B) comprenant un agent oxydant dans un milieu approprié pour la teinture.

D'autres objets de l'invention apparaîtront à la lecture de la description et des exemples qui suivent.

Les sels d'acide utilisés selon l'invention sont choisis de préférence parmi les chlorhydrates, les sulfates, les bromhydrates et les tartrates.

Le 3-méthyl para-aminophénol, le 2-méthyl para-aminophénol et le 2-hydroxyméthyl para-aminophénol ou leurs sels, sont présents à une concentration totale de 0.01 à 4% en poids par rapport au poids total de la composition tinctoriale, et de préférence de 0.1 à 2% en poids.

Les 2-méthyl 5-aminophénols de formule (I) ainsi que leurs sels, représentent au total de 0,01 à 5% en poids du poids total de la composition tinctoriale, et de préférence de 0,2 à 3,5% en poids.

Les métaphénylènediamines de formule (II) ci-dessus représentent de 0.002 à 2% en poids par rapport au poids total de la composition tinctoriale, et de préférence de 0,05 à 1% en poids.

L'ensemble précurseurs de colorants d'oxydation et coupleurs selon l'invention, représente de 0,1 à 10% en poids, et de préférence de 0,4 à 5% en poids, par rapport au poids total de la composition.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

La composition (A), qui renferme l'association des colorants telle-que décrite ci-dessus, peut avoir un pH compris entre 3 et 10,5, qui peut être ajusté à la valeur choisie au moyen d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule : 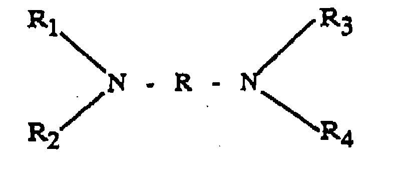 dans laquelle :
R est un reste propylène éventuellement substitué par un groupement hydroxyle ou un radical alkyle en C₁-C₄,
R₁, R₂, R₃ et R₄, simultanément ou indépendamment l'un de l'autre, représentent un atome d'hydrogène, un radical alkyle en C₁-C₄ ou hydroxyalkyle en C₁-C₄;
ou d'agents acidifiants classiques, tels que les acides minéraux ou organiques, par exemple les acides chlorhydrique, tantrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisant bien connus de l'état de la technique, tels que décrits ci-dessus.

La composition oxydante (B) est de préférence constituée par une solution d'eau oxygénée.

Selon un mode de réalisation préféré du procédé de teinture de l'invention, on mélange au moment de l'emploi la composition tinctoriale (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on les rince, les lave au shampooing, on les rince à nouveau et on les sèche.

Selon l'invention, les compositions tinctoriales peuvent contenir, en plus des colorants définis ci-dessus, d'autres coupleurs et/ou des colorants directs, notamment en vue de nuancer ou d'enrichir en reflets les colorations apportées par les précurseurs de colorants d'oxydation.

Ces coupleurs sont bien connus en eux-mêmes et sont choisis parmi les composés benzéniques portant au moins 2 substitutions hydroxy et/ou amino éventuellement modifié en position méta l'une par rapport à l'autre et différents des 2-méthyl 5-aminophénols de formule (I) et des métaphénylènediamines de formule (II); l'α-naphtol; les dérivés indoliques; les coupleurs possédant un groupement méthylène actif, tels que les composés β-cétoniques; les pyrazolones; ainsi que leurs sels.

Les colorants directs sont de préférence des colorants azoïques, anthraquinoniques ou des dérivés nitrés de la série benzénique.

Les compositions tinctoriales conformes à l'invention contiennent également dans leur forme de réalisation préférée, des agents tensioactifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les alkylpolyglycosides, les sels d'ammonium quaternaire, tels que le bromure de triméthylcétylammonium, le bromure de cétylpyridinium, les éthanolamides d'acides gras éventuellement oxyéthylénés, les acides, alcools et amines polyoxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0.5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄. tels que l'éthanol et l'isopropanol; le glycérol, les glycols ou éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention, peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0.1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique.

Ces agents antioxydants sont présents dans la composition dans des proportions comprises entre 0.05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. Ces compositions peuvent être conditionnées sous pression en flacons aérosols en présence d'un agent propulseur et former des mousses.

Les exemples qui suivent sont destinés à illustrer l'invention sans pour autant présenter un caractère limitatif.

### TEINTURE A pH ACIDE

### EXEMPLES 1 et 2

On prépare la composition tinctoriale suivante :

| | |
|---|---|
| . Alcool oléique polyglycérolé à 2 moles de glycérol | 4,0 g |
| . Alcool oléique polyglycérolé à 4 moles de glycérol (78% de MA) | 5,7 g MA |
| Acide oléique | 3,0 g |
| . Amine oléique oxyéthylénée à 2 moles d'oxyde d'éthylène, vendue sous la dénomination ETHOMEEN O12 par la Société AKZO | 7,0 g |
| . Laurylamino succinamate de diéthylaminopropyle, sel de sodium à 55% de MA | 3,0 g MA |
| . Alcool oléique | 5,0 g |
| . Diéthanolamide d'acide oléique | 12,0 g |
| . Propylèneglycol | 3,5 g |
| . Alcool éthylique | 7,0 g |
| . Dipropylèneglycol | 0,5 g |
| . Monométhyléther de propylèneglycol | 9,0 g |
| . Métabisulfite de sodium en solution aqueuse à 35% de MA | 0.46 g MA |
| . Acétate d'ammonium | 0,8 g |
| . Antioxydant séquestrant qs | |
| . Parfum, conservateur qs | |
| . Monoéthanalamine | 0,8 g |
| . Colorants | x g |
| · Eau déminéralisée qsp | 100 g |

Le pH de la composition tinctoriale de l'exemple 1 avant mélange est de 8,6.

Le pH de la composition tinctoriale de l'exemple 2 avant mélange est de 8,7.

Au moment de l'emploi, on mélange la composition tinctoriale poids pour poids avec de l'eau oxygénée titrant 20 volumes (6% en poids) et dont le pH est ajusté à 1.4 par 1,7 g d'acide ortho-phbsphorique pour 100 g d'eau oxygénée.

On obtient un mélange dont le pH est indiqué dans le tableau ci-après.

On applique ce mélange sur les cheveux pendant 30 minutes. Après rinçage, lavage au shampooing, rinçage et séchage, les cheveux sont teints dans les nuances indiquées dans le tableau ci-après.

| Exemple | 1 | 2 |
|---|---|---|
| 3-méthyl p-aminophénol | 1 g | 0,5 g |
| 2-méthyl 5-méthylaminophénol | 0,8 g | |
| 2-méthyl 5-éthylaminophénol | | 0,4 g |
| 1-β-hydroxyéthyloxy 2,4-diaminobenzène, dichlorhydrate | 0,6 g | |
| 1-(1,2-dihydroxypropyloxy)2.4-diaminobenzène, dichlorhydrate | | 0,3 g |
| pH du mélange | 6,7 | c |

| NUANCE OBTENUE: | | |
|---|---|---|
| sur cheveux gris naturels à 90% blancs | Blond beige très nacré | Blond clair beige doré nacré |

## Revendications

1. Composition tinctoriale pour fibres kératiniques, en particulier pour fibres kératiniques humaines telles que les cheveux, **caractérisée par le fait qu'**elle comprend, dans un milieu approprié pour la teinture :
- au moins un précurseur de colorant d'oxydation choisi parmi le 3-méthyl para-aminophéol, le 2-méthyl para-aminophénol et le 2-hydroxyméthyl para-aminophénol, et leurs sels d'addition avec un acide:
- au moins un coupleur choisi parmi les 2-méthyl 5-aminophénols de formule (I): 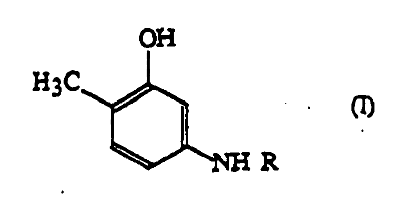
dans laquelle R désigne un radical méthyle ou éthyle, ou un groupement γ-hydroxyprople, ainsi que leurs sels d'addition avec un acide: et
au moins, à titre de coupleur additionnel, une métaphénylènediamine de formule (II) suivante : 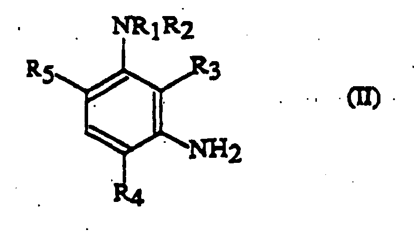 dans laquelle :
R₁ et R₂ désignent, indépendamment run de l'autre, un atome d'hydrogène ou un groupement alkyle en C₁-C₄ ou hydroxyalkyle en C₁-C₄:
R₃ désigne un atome d'hydrogène ou un groupement alkyle ou alcoxy en C₁-C₄:
R₄ désigne un atome d'hydrogène ou un groupement alkyle en C₁-C₄ ou hydroxyalcoxy en C₁-C₄, ou alcoxy en C₁-C₄;
R₅ désigne un atome d'hydrogène ou un groupement alkyle en C₁-C₄, alcoxy en C₁-C₄, hydroxyalkyle en C₁-C₄, hydroxyalcoxy en C₁-C₄, polyhydroxyalcoxy en C₂-C₄, un atome d'halogène, ou un groupement carboxyalcoxy en C₁-C₄, 2',4'-dlaminophénoxyalcoxy en C₁-C₄, ou aminoalcoxy en C₁-C₄;
sous réserve que si R₅ désigne un groupement carboxyalcoxy ou 2',4'-diaminophénoxyalcoxy, alors R₁, R₂, R₃ et R₄ désignent un atome d'hydrogène, si R₅ désigne un groupement alkyle en C₁-C₄ et R₁, R₂ et R₃ désignent un atome d'hydrogène, R₄ ne désigne pas un groupement alcoxy en C₁-C₄ ou hydroxyalkoxy en C₁-C₄, et si R₁, R₂ et R₄ désignent un atome d'hydrogène, R₃ et R₅ ne désignent pas simultanément un radical méthoxy,
ou l'un de ses sel d'addition avec un acide.

2. Composition tinctoriale selon la revendication 1, **caractérisée par le fait que** le précurseur de colorant d'oxydation de type para est le 3-méthyl para-aminophénol ou l'un de ses sels d'addition avec un acide.

3. Composition tinctoriale selon l'une quelconque des revendications 1 à 2, **caractérisée par le fait que** la métaphénylènediamine de formule (II) est choisie parmi le 1-β-hydroxyéthyloxy 2,4-diaminobenzène, le 1-(1,2-dihydroxypropyloxy)2,4-diamincbenzène, le 1-[2',4'-diaminophénoxypropyloxy]2,4-diaminobenzène, le 1-méthoxy 2-amino 4-β-hydroxyéthylaminobenzène, l'acide 2,4-diaminophénoxyacétique et le 4,6-bis-(2-hydroxyéthyloxy)1,3-diaminobenzène, ainsi que leurs sels d'addition avec un acide.

4. Composition tinctoriale selon l'une quelconque des revendications 1 à 3, **caractérisée par le fait que** les sels d'addition avec un acide sont choisis parmi les chlorhydrates, les sulfates, les bromhydrates et les tartrates.

5. Composition tinctoriale selon l'une quelconque des revendications 1 à 4, **caractérisée par le fait que** le 3-méthyl paraaminophénol, le 2-méthyl para-aminophénol et le 2-hydroxyméthyl para-aminophénol ou leurs sels, sont présents dans une concentration totale de 0.01 à 4% en poids, et de préférence de 0,1 à 2% en poids par rapport au poids total de la composition.

6. Composition tinctoriale selon l'une quelconque des revendications 1 à 5, **caractérisée par le fait que** les 2-méthyl 5-aminophénols de formule (I) ou leurs sels, sont présents à une concentration totale de 0.01 à 5% en poids, et de préférence de 0,2 à 3,5% en poids par rapport au poids total de la composition.

7. Composition tinctoriale selon l'une quelconque des revendications 1 à 6, **caractérisée par le fait que** la métaphénylènediamine de formule (II) ou ses sels est présente à une concentration de 0,002 à 2% en poids, et de préférence de 0,05 à 1% en poids par rapport au poids total de la composition.

8. Composition tinctoriale selon l'une quelconque des revendications 1 à 7, **caractérisée par le fait que** les précurseurs de colorants d'oxydation et les coupleurs sont présents dans une concentration totale de 0,1 à 10% en poids, et de préférence de 0,4 à 5% en poids, par rapport au poids total de la composition.

9. Composition tinctoriale selon l'une quelconque des revendications 1 à 8, **caractérisée par le fait qu'**elle a un pH compris entre 3 et 10,5.

10. Composition tinctoriale selon l'une quelconque des revendications 1 à 9, **caractérisée par le fait qu'**elle contient d'autres coupleurs choisis parmi les composés benzéniques portant au moins 2 substitutions hydroxy et/ou amino éventuellement modifié en position méta l'une par rapport à l'autre et différents des 2-méthyl 5-aminophénols de formule (I) et des métaphénylènediamines de formule (II): l'α-naphtol: les dérivés indoliques; les composés β-cétoniques; les pyrazolones; ainsi que leurs sels.

11. Composition tinctoriale selon rune quelconque des revendications 1 à 10, **caractérisée par le fait qu'**elle contient en outre des colorants directs choisis parmi les colorants azoïques, anthraquinoniques et les dérivés nitrés de la série benzénique.

12. Composition tinctoriale selon l'une quelconque des revendications 1 à 11, **caractérisée par le fait qu'**elle contient en outre au moins un adjuvant choisi parmi les agents tensio-actifs anioniques, cationiques, non-ioniques, amphotères ou leurs mélanges en des proportions comprises entre 0,5 et 55% en poids, les solvants organiques en des proportions comprises entre 1 et 40% en poids, les agents épaississants en des proportions comprises entre 0,1 et 5% en poids, les agents antioxydants dans des proportions comprises entre 0,05 et 1,5% en poids, les proportions étant calculées par rapport au poids total de la composition, les agents de pénétration, les agents séquestrants, les parfums, les tampons, les agents dispersants, les agents de conditionnement, les agents filmogènes, les agents conservateurs et les agents opacifiants.

13. Agent de teinture des fibres kératiniques et en particulier des fibres kératiniques humaines telles que les cheveux, **caractérisé par le fait qu'**il comporte au moins deux composants : un composant (A) constitué par une composition de teinture selon l'une quelconque des revendications 1 à 12, et un composant (B) comprenant un agent oxydant dans un milieu approprié pour la teinture.

14. Agent de teinture selon la revendication 13, **caractérisé par le fait que** ragent oxydant est choisi parmi le peroxyde d'hydrogène, le peroxyde d'urée, les bromates de métaux alcalins, les perborates et les persulfates.

15. Procédé de teinture des fibres kératiniques et en particulier des fibres kératiniques humaines telles que les cheveux, **caractérisé par le fait qu'**il consiste à appliquer sur les fibres une composition tinctoriale (A) selon l'une quelconque des revendications 1 à 12, et à révéler la couleur en milieu acide ou alcalin à l'aide d'un agent oxydant ajouté juste au moment de l'emploi à cette composition ou présent dans une composition (B) appliquée simultanément ou séquentiellement de façon séparée.

16. Procédé de teinture selon la revendication 15, **caractérisé par le fait qu'**on mélange au moment de l'emploi la composition tinctoriale selon l'une quelconque des revendications 1 à 12 avec une solution oxydante en une quantité suffisante pour développer une coloration, puis on applique le mélange obtenu sur les fibres, on laisse poser pendant 5 à 40 minutes, de préférence 15 à 30 minutes, puis on rince, on lave au shampooing, on rince à nouveau et on sèche.

17. Dispositif à plusieurs compartiments ou kit de teinture, **caractérisé par le fait qu'**il comporte au moins deux compartiments dont un premier compartiment renferme la composition (A) telle que définie dans l'une quelconque des revendications 1 à 12, et le second compartiment renferme la composition (B) comprenant un agent oxydant dans un milieu approprié pour la teinture..

## Claims

1. Dyeing composition for keratinous fibres, in particular for human keratinous fibres such as hair, **characterized in that** it comprises, in a suitable medium for dyeing:
- at least one oxidation dye precursor chosen from 3-methyl-para-aminophenol, 2-methyl-para-aminophenol and 2-hydroxymethyl-para-aminophenol, and their addition salts with an acid;
- at least one coupling agent chosen from the 2-methyl-5-aminophenols of formula (I): 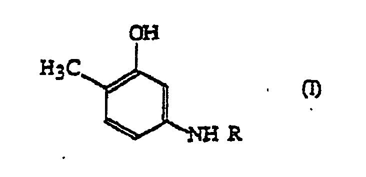 in which R denotes a methyl or ethyl radical or a γ-hydroxypropyl group; and their addition salts with an acid; and
- as additional coupling agent, at least one meta-phenylenediamine of formula (II) below: 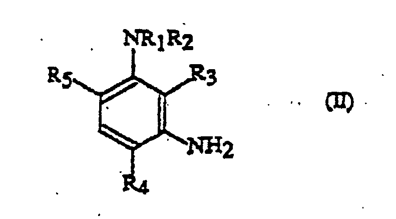
in which:
R₁ and R₂ denote, independently of each other, a hydrogen atom or a C₁-C₄ alkyl or C₁-C₄ hydroxyalkyl group;
R₃ denotes a hydrogen atom or a C₁-C₄ alkyl or alkoxy group;
R₄ denotes a hydrogen atom or a C₁-C₄ alkyl or C₁-C₄ hydroxyalkoxy or C₁-C₄ alkoxy group;
R₅ denotes a hydrogen atom or a C₁-C₄ alkyl, C₁-C₄ alkoxy, C₁-C₄ hydroxyalkyl, C₁-C₄ hydroxyalkoxy or C₂-C₄ polyhydroxyalkoxy group, a halogen atom or a C₁-C₄ carboxyalkoxy, C₁-C₄ 2',4'-diaminophenoxyalkoxy or C₁-C₄ aminoalkoxy group;
with the proviso that if R₅ denotes a carboxyalkoxy or 2',4'-diaminophenoxyalkoxy group, then R₁, R₂, R₃ and R₄ denote a hydrogen atom, if R₅ denotes a C₁-C₄ alkyl group and R₁, R₂ and R₃ denote a hydrogen atom, R₄ does not denote a C₁-C₄ alkoxy group or a C₁-C₄ hydroxyalkoxy group, and if R₁, R₂ and R₄ denote a hydrogen atom, R₃ and R₅ do not simultaneously denote a methoxy radical,
or one of its addition salts with an acid.

2. Dyeing composition according to Claim 1, **characterized in that** the oxidation dye precursor of para type is 3-methyl-para-aminophenol or one of its addition salts with an acid.

3. Dyeing composition according to either one of Claims 1 and 2, **characterized in that** the meta-phenylenediamine of formula (II) is chosen from: 1-β-hydroxyethyloxy-2,4-diaminobenzene, 1-(1,2-dihydroxypropyloxy)-2,4-diaminobenzene, 1-(2',4'-diaminophenoxypropyloxy)-2,4-diaminobenzene, 1-methoxy-2-amino-4-β-hydroxyethylaminobenzene, 2,4-diaminophenoxyacetic acid and 4,6-bis(2-hydroxyethyloxy)-1,3-diaminobenzene, and their addition salts with an acid.

4. Dyeing composition according to any one of Claims 1 to 3, **characterized in that** the addition salts with an acid are chosen from the hydrochlorides, sulphates, hydrobromides and tartrates.

5. Dyeing composition according to any one of Claims 1 to 4, **characterized in that** 3-methyl-para-aminophenol, 2-methyl-para-aminophenol and 2-hydroxymethyl-para-aminophenol or their salts are present in a total concentration of 0.01 to 4% by weight and preferably from 0.1 to 2% by weight relative to the total weight of the composition.

6. Dyeing composition according to any one of Claims 1 to 5, **characterized in that** the 2-methyl-5-aminophenols of formula (I) or their salts are present at a total concentration of 0.01 to 5% by weight and preferably from 0.2 to 3.5% by weight relative to the total weight of the composition.

7. Dyeing composition according to any one of Claims 1 to 6, **characterized in that** the meta-phenylenediamine of formula (II) or its salts is present at a concentration of 0.002 to 2% by weight and preferably from 0.05 to 1% by weight relative to the total weight of the composition.

8. Dyeing composition according to any one of Claims 1 to 7, **characterized in that** the oxidation dye precursors and the coupling agents are present in a total concentration of 0.1 to 10% by weight and preferably from 0.4 to 5% by weight relative to the total weight of the composition.

9. Dyeing composition according to any one of Claims 1 to 8, **characterized in that** it has a pH between 3 and 10.5.

10. Dyeing composition according to any one of Claims 1 to 9, **characterized in that** it contains other coupling agents chosen from benzene compounds bearing at least two hydroxyl and/or optionally modified amino substitutions in the meta-position with respect to each other and which are different from the 2-methyl-5-aminophenols of formula (I) and from the meta-phenylenediamines of formula (II); α-naphthol; indole derivatives; β-keto compounds; pyrazolones; as well as their salts.

11. Dyeing composition according to any one of Claims 1 to 10, **characterized in that** it additionally contains direct dyes chosen from azo and anthraquinone dyes and nitro derivatives from the benzene series.

12. Dyeing composition according to any one of Claims 1 to 11, **characterized in that** it additionally contains at least one adjuvant chosen from anionic, cationic, nonionic and amphoteric surface-active agents or their mixtures in proportions between 0.5 and 55% by weight, organic solvents in proportions between 1 and 40% by weight, thickening agents in proportions between 0.1 and 5% by weight, and antioxidants in proportions between 0.05 and 1.5% by weight, the proportions being calculated relative to the total weight of the composition, penetration agents, sequestering agents, perfumes, buffers, dispersing agents, conditioning agents, film-forming agents, preservatives and opacifying agents.

13. Dyeing agent for keratinous fibres and in particular for human keratinous fibres such as hair, **characterized in that** it contains at least two components: a component (A) consisting of a dyeing composition according to any one of Claims 1 to 12, and a component (B) comprising an oxidizing agent in a suitable medium for dyeing.

14. Dyeing agent according to Claim 13, **characterized in that** the oxidizing agent is chosen from hydrogen peroxide, urea peroxide, alkali metal bromates, perborates and persulphates.

15. Process for dyeing keratinous fibres and in particular human keratinous fibres such as hair, **characterized in that** it consists in applying to the fibres a dyeing composition (A) according to any one of Claims 1 to 12, and in developing the colour in an acidic or alkaline medium using an oxidizing agent which is added just at the time of use to this composition or which is present in a composition (B) which is applied simultaneously or sequentially in a separate manner.

16. Process for dyeing according to Claim 15, **characterized in that** the dyeing composition according to any one of Claims 1 to 12 is mixed at the moment of use with an oxidizing solution in a sufficient amount to develop a colouration, the mixture obtained is then applied to the fibres, it is left to stand for 5 to 40 minutes, preferably 15 to 30 minutes, and then the fibres are rinsed, washed with shampoo, rinsed again and dried.

17. Dyeing kit or multicompartment device, **characterized in that** contains at least two compartments, a first compartment of which contains the composition (A) as defined in any one of Claims 1 to 12, and the second compartment of which contains the composition (B) comprising an oxidizing agent in a suitable medium for dyeing.

## Patentansprüche

1. Farbstoffzusammensetzung für Keratinfasern, insbesondere menschliche Keratinfasern, wie Haaren, **dadurch gekennzeichnet, dass** sie in einem zur Färbung geeigneten Medium umfasst:
- mindestens einen Oxidationsfärbemittelvorläufer, ausgewählt aus 3-Methyl-p-aminophenol, 2-Methyl-p-aminophenol und 2-Hydroxymethyl-p-aminophenol und deren Säureadditionssalzen;
- mindestens einen Kuppler, ausgewählt aus den 2-Methyl-5-aminophenolen der Formel (I): 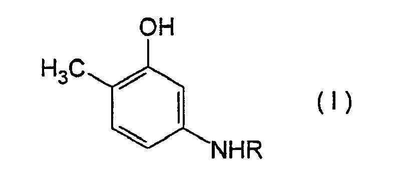 worin R einen Methyl- oder Ethylrest oder eine γ-Hydroxypropylgruppe bedeutet, sowie deren Säureadditionssalzen; und
- mindestens ein m-Phenylendiamin der folgenden Formel (II): 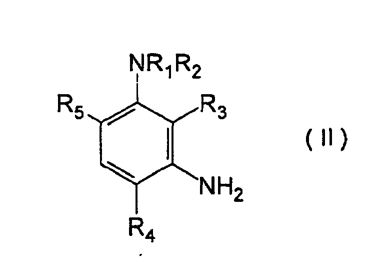 als zusätzlichen Kuppler,
worin gilt:
R₁ und R₂ bedeuten, unabhängig voneinander, ein Wasserstoffatom oder eine C₁₋₄-Alkyl- oder eine C₁₋₄-Hydroxyalkylgruppe;
R₃ bedeutet ein Wasserstoffatom oder eine C₁₋₄-Alkyl- oder eine C₁₋₄-Alkoxygruppe;
R₄ bedeutet ein Wasserstoffatom oder eine C₁₋₄-Alkyl- oder C₁₋₄-Hydroxyalkoxy- oder eine C₁₋₄-Alkoxygruppe;
R₅ bedeutet ein Wasserstoffatom oder eine C₁₋₄-Alkyl-, C₁₋₄-Alkoxy-, C₁₋₄-Hydroxyalkyl-, C₁₋₄-Hydroxyalkoxy-, C₂₋₄-Polyhydroxyalkoxygruppe, ein Halogenatom oder eine C₁₋₄-Carboxyalkoxy-, C₁₋₄-2'_{,}4'-Diaminophenoxyalkoxy- oder eine C₁₋₄-Aminoalkoxygruppe;
mit der Massgabe, dass, wenn R₅ eine Carboxyalkoxy- oder eine 2',4'-Diaminophenoxyalkoxygruppe bedeutet, R₁, R₂, R₃ und R₄ dann ein Wasserstoffatom bedeuten, wenn R₅ eine C₁₋₄-Alkylgruppe und R₁, R₂ und R₃ ein Wasserstoffatom bedeuten, R₄ dann keine C₁₋₄-Alkoxy- oder C₁₋₄-Hydroxyalkoxygruppe bedeutet, und wenn R₁, R₂ und R₄ ein Wasserstoffatom bedeuten, R₃ und R₅ dann nicht gleichzeitig einen Methoxyrest bedeuten,
oder eines seiner Additionssalze mit einer Säure.

2. Farbstoffzusammensetzung gemäss Anspruch 1, **dadurch gekennzeichnet, dass** der Oxidationsfärbemittelvorläufer vom p-Typ 3-Methyl-p-aminophenol oder eines seiner Säureadditionssalze ist.

3. Farbstoffzusammensetzung gemäss irgendeinem der Ansprüche 1 bis 2, **dadurch gekennzeichnet, dass** das m-Phenylendiamin der Formel (II) aus 1-β-Hydroxyethyloxy-2,4-diaminobenzol, 1-(1,2-Dihydroxypropyloxy)-2,4-diaminobenzol, 1-[2',4'-Diaminophenoxypropyloxy]-2,4-diaminobenzol, 1-Methoxy-2-amino-4-β-hydroxyethylaminobenzol, 2,4-Diaminophenoxyessigsäure und aus 4,6-Bis(2-hydroxyethyloxy)-1,3-diaminobenzol sowie aus deren Säureadditionssalzen ausgewählt ist.

4. Farbstoffzusammensetzung gemäss einem der Ansprüche 1 bis 3, **dadurch gekennzeichnet, dass** die Säureadditionssalze aus Hydrochloriden, Sulfaten, Hydrobromiden und aus Tartraten ausgewählt sind.

5. Farbstoffzusammensetzung gemäss einem der Ansprüche 1 bis 4, **dadurch gekennzeichnet, dass** das 3-Methyl-p-aminophenol, 2-Methyl-p-aminophenol und das 2-Hydroxymethyl-p-aminophenol oder deren Salze in einer Gesamtkonzentration von 0,01 bis 4 Gew.% und vorzugsweise von 0,1 bis 2 Gew.% vorhanden sind, bezogen auf das Gesamtgewicht der Zusammensetzung.

6. Farbstoffzusammensetzung gemäss einem der Ansprüche 1 bis 5, **dadurch gekennzeichnet, dass** die 2-Methyl-5-aminophenole der Formel (I) oder deren Salze in einer Gesamtkonzentration von 0,01 bis 5 Gew.% und vorzugsweise von 0,2 bis 3,5 Gew.% vorhanden sind, bezogen auf das Gesamtgewicht der Zusammensetzung.

7. Farbstoffzusammensetzung gemäss einem der Ansprüche 1 bis 6, **dadurch gekennzeichnet, dass** das m-Phenylendiamin der Formel (II) oder seine Salze in einer Konzentration von 0,002 bis 2 Gew.% und vorzugsweise von 0,05 bis 1 Gew.% vorhanden sind, bezogen auf das Gesamtgewicht der Zusammensetzung.

8. Farbstoffzusammensetzung gemäss einem der Ansprüche 1 bis 7, **dadurch gekennzeichnet, dass** die Oxidationsfärbemittelvorläufer und die Kuppler in einer Gesamtkonzentration von 0,1 bis 10 Gew.% und vorzugsweise von 0,4 bis 5 Gew.% vorhanden sind, bezogen auf das Gesamtgewicht der Zusammensetzung.

9. Farbstoffzusammensetzung gemäss einem der Ansprüche 1 bis 8, **dadurch gekennzeichnet, dass** sie einen pH-Wert von 3 bis 10,5 aufweist.

10. Farbstoffzusammensetzung gemäss einem der Ansprüche 1 bis 9, **dadurch gekennzeichnet, dass** sie weitere Kuppler enthält, ausgewählt aus benzoischen Verbindungen, die mindestens zwei Hydroxy- und/oder Aminosubstitutionen aufweisen, die in m-Position zueinander gegebenenfalls modifiziert sind und sich von den 2-Methyl-5-aminophenolen der Formel (I) und den m-Phenylendiaminen der Formel (II) unterscheiden, aus α-Naphthol, Indolderivaten, β-Ketoverbindungen, Pyrazolonen sowie aus deren Salzen.

11. Farbstoffzusammensetzung gemäss einem der Ansprüche 1 bis 10, **dadurch gekennzeichnet, dass** sie ausserdem Direktfarbstoffe enthält, ausgewählt aus Azo-, Anthrachinonfarbstoffen und aus nitrierten Derivaten der Benzolreihe.

12. Farbstoffzusammensetzung gemäss einem der Ansprüche 1 bis 11, **dadurch gekennzeichnet, dass** sie ausserdem mindestens einen Hilfsstoff enthält, ausgewählt aus anionischen, kationischen, nicht-ionischen und amphoteren Mitteln oder aus deren Mischungen in Mengenanteilen von 0,5 bis 55 Gew.%, aus organischen Lösungsmitteln in Mengenanteilen von 1 bis 40 Gew.%, aus Verdickungsmitteln in Mengenanteilen von 0,1 bis 5 Gew.%, aus Antioxidantien in Mengenanteilen von 0,05 bis 1,5 Gew.%, wobei die Mengenanteile unter Bezug auf das Gesamtgewicht der Zusammensetzung berechnet sind, aus Eindringmitteln, Sequestriermitteln, Parfümprodukten, Puffermitteln, Dispergiermitteln, Konditioniermitteln, filmbildenden Mitteln, Konservierungsmitteln und aus Trübungsmitteln.

13. Mittel zur Färbung von Keratinfasern und insbesondere menschlichen Keratinfasern, wie Haaren, **dadurch gekennzeichnet, dass** es mindestens zwei Bestandteile umfasst: einen Bestandteil (A) aus einer Zusammensetzung zur Färbung gemäss einem der Ansprüche 1 bis 12 und einen Bestandteil (B), der ein Oxidationsmittel in einem zur Färbung geeigneten Medium enthält.

14. Mittel zur Färbung gemäss Anspruch 13, **dadurch gekennzeichnet, dass** das Oxidationsmittel aus Wasserstoffperoxid, Harnstoffperoxid, Alkalimetallbromaten, Perboraten und aus Persulfaten ausgewählt ist.

15. Verfahren zur Färbung von Keratinfasern und insbesondere menschlichen Keratinfasern, wie Haaren, **dadurch gekennzeichnet, dass** man auf die Fasern eine Farbstoffzusammensetzung (A) gemäss einem der Ansprüche 1 bis 12 aufbringt und einwirken lässt und man die Farbe in saurem oder alkalischem Medium mit einem Oxidationsmittel sich entwickeln lässt, das kurz vor der Anwendung dieser Zusammensetzung zugefügt wird oder in einer Zusammensetzung (B) vorliegt, die gleichzeitig oder getrennt voneinander aufgebracht wird.

16. Verfahren zur Färbung gemäss Anspruch 15, **dadurch gekennzeichnet, dass** man zum Zeitpunkt der Anwendung die Farbstoffzusammensetzung gemäss einem der Ansprüche 1 bis 12 mit einer oxidierenden Lösung in einer zur Entwicklung einer Färbung ausreichenden Menge vermischt, die erhaltene Mischung auf die Fasern aufbringt, das Ganze 5 bis 40 min und vorzugsweise 15 bis 30 min lang verweilen lässt, dann spült, unter Shamponieren wäscht, erneut spült und trocknet.

17. Vorrichtung aus mehreren Teilen oder Kit zur Färbung, **dadurch gekennzeichnet, dass** sie mindestens zwei Teile aufweist, deren erster Teil die in einem jeden der Ansprüche 1 bis 12 definierte Zusammensetzung (A) und deren zweiter Teil die Zusammensetzung (B) umfasst, die ein oxidierendes Mittel in einem zur Färbung geeigneten Medium enthält.
